# EUROPEAN PATENT SPECIFICATION

(11) **EP 2 635 264 B1**
(45) Date of publication and mention of the grant of the patent: **30.05.2018**
(21) Application number: 11785049.5
(22) Date of filing: 04.11.2011
(51) Int. Cl.: A61K 9/20, A61P 1/10, A61K 31/765

(54) **FORMULATIONS COMPRISING POLYETHYLENE GLYCOL**
FORMULIERUNGEN MIT POLYETHYLENGLYKOL
FORMULATIONS COMPORTANT DU POLYÉTHYLÈNE GLYCOL

(30) Priority: 06.05.2011 GB 201107626; 09.03.2011 GB 201104049; 04.11.2010 GB 201018647; 10.11.2010 US 412109 P
(43) Date of publication of application: 11.09.2013
(73) Proprietor: Norgine B.V., 1101 CA Amsterdam ZO (NL)
(72) Inventor: STEIN, Peter, Uxbridge Middlesex UB9 6NS (GB); COX, Ian, Hengoed Mid-Glamorgan CF82 8SJ (GB); SMITH, Samuel, Hengoed Mid-Glamorgan CF82 8SJ (GB); JONES, Leighton, Hengoed Mid-Glamorgan CF82 8SJ (GB); PLESSL, Jörg, Hengoed Mid-Glamorgan CF82 8SJ (GB)
(74) Representative: Abel & Imray
(86) International application number: PCT/GB2011/001560
(87) International publication number: WO 2012/059724

(56) References cited:
- WO-A1-97/03685
- WO-A1-2005/102364
- WO-A1-2006/122104

## Description

The present invention relates to solid formulations, particularly to chewable or suckable solid formulations (for example tablets), comprising polyethylene glycol (PEG) and mannitol or other solid. In particular, the formulations are solid, low-dosage forms for chronic consumption by healthy subjects to maintain gastro-intestinal health and prevent gastro-intestinal disorders.

PEG is well known in pharmaceuticals either in small amounts at low molecular weight (eg PEG 400) as an excipient, or at high doses in aqueous solution at higher molecular weight (eg PEG 3350 or 4000Da) as an active agent for use as a laxative or bowel preparation, often in combination with other osmotic agents or electrolytes. Various such PEG / electrolyte products are on the market in many countries. An example of such a product is MOVICOL (registered trademark of the Norgine group of companies, and marketed in the UK by Norgine Limited, Norgine House, Widewater Place, Moorhall Road, Harefield, Uxbridge, Middlesex UB9 6NS, United Kingdom). MOVICOL is provided in a sachet containing 13.8g powder for making up into an oral solution. Each sachet contains: 13.1250g Macrogol (polyethylene glycol (PEG)) 3350, 0.3507g sodium chloride, 0.1785g sodium bicarbonate and 0.0466g potassium chloride. This is the standard dose of MOVICOL. It also contains flavouring and sweetener. MOVICOL has been on the market since 1995.

As well as in patients suffering from constipation, such high doses have also been shown to have a laxative effect in healthy volunteers. For example Flourie *et al.* (Flourie, B et al., Gastroenterol. Clin. Biol., 1994, 18, A108) showed that stool weight and stool frequency were significantly increased in healthy subjects taking an aqueous solution of 26g per day of PEG with electrolytes (sodium chloride, sodium bicarbonate and potassium chloride).

Hudziak *et al.* (Hudziak, H. et al., Gastroenterol. Clin. Biol., 1996, 20, 418-423) showed that healthy subjects taking an aqueous solution of 20g of PEG 4000 per day (without accompanying electrolytes) had a significantly increased stool frequency. Mean stool weight was also shown to be increased.

Sometimes PEG and electrolyte solutions comprise, in addition to PEG, another osmotic agent to increase the laxative capability of the solution. For example, Bernier and Donazzolo (Bernier, J-J., and Donazzolo, Y., Gastroenterol. Clin. Biol., 1997, 21, 7-11) reported that consumption of an aqueous solution of 5.9g per day of PEG 3350 in the presence of electrolytes (5.9g PEG per day with 146mg sodium chloride, 568mg sodium sulphate, 75mg potassium chloride and 168mg sodium bicarbonate) for a total period of seven days led to stool softening in healthy subjects. However, the presence of sodium sulphate (another osmotic agent) in the preparations used in this study renders the effect of the PEG, and therefore the results described therein, uncertain. Furthermore, sodium sulphate imparts a taste that is generally regarded as unpleasant.

In the vast majority of published work, including those mentioned above, regarding PEG-based products for oral consumption, the PEG is taken as a solution/suspension in water. Taking compositions as solutions or suspensions is in many cases less convenient than taking a solid composition, as solutions and suspensions require the subject to carry a larger quantity of composition with them, or else require the subject to make use of a vessel and a source of liquid. Particularly for compositions that may be taken several times per day and/or at a time of the subject's choosing, solid compositions offer many advantages to a subject.

Solid PEG products for consumption in a solid dosage formulation have been described in some patent specifications. For example, in WO2005/102364, there is described a solid pharmaceutical composition comprising PEG and electrolytes for treating constipation, faecel impaction, faecal retention, intestinal gas and cramping, flatulence, or for cleansing the colon, wherein the PEG makes up from 80 to 99.5% by weight of the composition. In WO2006/122104, there are described possible ingredient ranges for a solid colonic purgative composition comprising PEG and various other ingredients. WO2006/122104 teaches a total daily dosage of PEG for "mild catharsis" of 10 to 100g of PEG.

To date, no PEG-based products for consumption in solid form have reached the market. There may be several reasons for this. Preparation of solid dosage forms that simultaneously have good structural integrity (i.e. sufficient hardness to hold together), but yet are comfortably chewable by a subject (i.e. a hardness that is not so high as to affect the subject's ease of taking the dosage) is not straightforward. Achieving the right degree of hardness requires particular consideration when the subject consuming the solid formulation is elderly or infirmed and who may suffer from impaired chewing capabilities. It is known to add excipients to solid dosage forms to assist in achieving satisfactory overall properties.

In addition, a solid dosage form must have good manufacturing properties (minimal capping or laminating of tablets, or sticking to tableting machinery) and excipients must not impart an unpleasant taste or mouthfeel to the formulation. Formulation of a PEG solid dosage form that has good manufacturing properties and good subject compliance is thus difficult.

Furthermore, the amount of PEG that would need to be consumed in order to treat constipation or to act as a bowel cleansing preparation would necessitate the consumption of an inconveniently large number of units of the solid dosage formulation in order for a patient to receive a sufficient amount of PEG to produce the requisite laxative effect.

In order for good compliance to be maintained, the subject must not experience discomfort or inconvenience when taking the dosage form. This is particularly the case where the subject is otherwise healthy (that is, for present purposes, has normal bowel movement) as such subjects are more likely to discontinue consumption of a composition that is either uncomfortable or inconvenient to take or which produces, following such consumption, gastrointestinal disturbances that they regard as unpleasant.

We have now surprisingly found that it is possible to formulate a solid formulation for oral administration as a solid comprising a non-laxative, relatively low (less than about 6g PEG per day) dose of PEG which is suitable for chronic consumption by healthy subjects.

Accordingly, the present invention provides a solid formulation for oral administration as a solid, comprising:
(a) 50 - 90 % w/w polyethylene glycol (PEG) having an average molecular weight within the range 2,000 to 10,000 Da; and
(b) 10 - 40 % w/w of a solid, selected from sorbitol, lactose, dextrates, cellulose, xylitol, mannitol.

The invention provides a solid formulation for oral administration as a solid, comprising:
(a) 50 - 90 % w/w polyethylene glycol (PEG) having an average molecular weight within the range 2,000 to 10,000 Da;
(b) 10 - 40 % w/w of a solid selected from sorbitol, lactose, dextrates, cellulose, xylitol, mannitol (sometimes referred to herein as "solid of component (b)"); and optionally
(c) q.s. to 100% w/w of further excipients such as flavourings, sweeteners and lubricants.
Herein, "% w/w" of a component is understood to mean the proportion, as a percentage, that the weight of the respective component makes up of the total weight of the solid formulation.

The present invention further provides a solid formulation for oral administration as a solid, comprising:
(a) 50 - 90 % w/w polyethylene glycol (PEG) having an average molecular weight within the range 2,000 to 10,000 Da; and
(b) 10 - 40 % w/w of a solid selected from the group: sorbitol, lactose, lactose and starch (e.g. a compound comprising lactose monohydrate and maize starch such as Starlac®), dextrates, cellulose (such as microcrystalline cellulose), xylitol, maltitol and mannitol.

Preferably, the solid of component (b) is greater than 10% w/w, preferably greater than 12% w/w, more preferably greater than 15% (e.g. 15% to 17%) of the solid formulation. Preferably the weight ratio of component (a) to component (b) is 1.25:1 to 9:1, preferably 2:1 to 7:1, preferably 4:1 to 6:1. In preferred embodiments, the ratio of component (a) to component (b) is approximately 5:1.

Preferably, the solid formulation is chewable and/or suckable. It may be a solid tablet, for example a chewable and/or suckable tablet.

It has surprisingly been found that a solid formulation of the invention is pleasantly chewable or suckable, has good taste, structural integrity and beneficial manufacturing properties. By "chewable" or "suckable" is meant herein that the solid formulation is for oral administration and is capable of being chewed or sucked in the mouth so that the first step in the digestive process starts in the buccal cavity. The formulations of the present invention are generally not intended to be swallowed whole without first being broken down, at least to some extent, in the buccal cavity prior to consumption. Herein, a powder formulation is not considered suitable for administration as a solid; especially those that require prior solution or suspension in a liquid (for example water) or delivery in an alternative medium or container. A formulation of the invention is preferably not effervescent in contact with water.

Formulations of the invention are preferably substantially free from electrolytes. Formulations of the invention that are substantially free from electrolytes may be particularly beneficial for those subjects who are healthy (that is, for present purposes, have normal bowel movement) but are hypertensive or otherwise following a low sodium diet. For example, they are preferably substantially free from sodium chloride, potassium chloride and sodium bicarbonate. They are preferably substantially free from sulphates or phosphates, for example, they are particularly preferred to be substantially free from sodium sulphate. Formulations of the invention are preferably substantially free from carbonates, bicarbonates, alkali metal ions and halide ions. Formulations of the present invention are most preferably substantially free from sodium, potassium, chloride, bicarbonate, carbonate and/or sulphate ions. In many instances, flavourings, lubricants and sweeteners may contain small amounts of electrolytes. Such amounts are not considered herein to be "substantial". Formulations of the invention are preferably substantially free from alginates and/or ascorbates and/or citrates. By "substantially free from" herein is also meant that the ingredient is not added to the formulation during preparation or manufacture.

In some embodiments, formulations of the present invention are substantially free from any osmotic agent other than PEG.

The polyethylene glycol (PEG) for use in solid formulations of the invention preferably has an average molecular weight (for example a weight average molecular weight), in Daltons, within the range 2,000 to 10,000, preferably 2,500 to 8,500, preferably 3,000 to 8,000, more preferably 3,000 to 6,000, more preferably 2,500 to 6,500, more preferably 2,500 to 4,500 for example 3,000 to 4,500, for example 3,000 to 4,100, for example 3,000 to 4,000. The PEG may have an average molecular weight within the range 6,000 to 10,000, for example 7,000 to 9,000. For example, the PEG may be, or comprise PEG 3,350, PEG 4,000 or PEG 8,000 as defined in national or regional pharmacopoeias. Further examples of suitable PEGs recognized in some national pharmacopoeias include Macrogols, for example Macrogol 4,000. Optionally, the PEG used in formulations of the invention may comprise two or more different PEG components. Optionally, the PEG used in formulations may have at least two differing average molecular weights. PEG of the relevant molecular weights in a form suitable for use in humans is available commercially.

In a preferred embodiment, PEG is present in the solid formulation in an amount of 60 to 90% w/w, preferably 70 to 90% w/w, more preferably 70 to 89 % w/w, for example 75 to 89 % w/w. In a further embodiment, PEG is present in an amount of 78 to 89 % w/w, for example 80 to 85% w/w, for example 81 to 85 % w/w, for example 80 to 84 % w/w, for example 82 to 84 % w/w. In a further embodiment, PEG is present in an amount of 50 to 80% w/w, for example 60 to 80% w/w, for example 70 to 80 % w/w, for example 70 to 79% w/w, for example 75 to 79% w/w.

The solid of component (b) of the invention is selected from the group consisting of sorbitol, lactose, lactose and starch, dextrates, cellulose (e.g. microcrystalline cellulose), xylitol, maltitol, mannitol. Lactose or similar ingredients may be present in hydrated form.

Where the solid of component (b) is lactose and starch, the lactose component may be in the form of a monohydrate. The starch component may be derived from any suitable source such as wheat starch, maize starch, potato starch and rice starch. The lactose component may make up 50% to 95% of the lactose/starch solid, for example 60% to 90%, e.g. 70 to 85% such as 85%.

Solids of component (b) of the invention for use in the present invention are preferably of a purity and grade suitable for consumption by e.g. humans.

The solid of component (b) makes up 10 to 40 % w/w of the solid formulation of the invention. In a preferred embodiment, the solid of component (b) makes up 10 to 30% w/w of the solid formulation, preferably greater than 10% w/w up to (and including) 30%w/w. For example, the solid of component (b) makes up 10 to 25% w/w, for example 10 to 20% w/w, preferably 12 to 20 % w/w, more preferably 12 to 19% w/w, 12 to 18% w/w, or 12 to 17 % w/w. For example, the solid of component (b) may make up 14 to 20% w/w, 14 to 19% w/w or 14 to 18% w/w 14 to 17% w/w of the solid formulation of the invention such as 15 to 16.5%w/w of the solid formulation of the invention.

Preferably, the solid of component (b) is mannitol. It has been found by the present inventors that a solid formulation comprising PEG and mannitol is more palatable than a solid formulation comprising PEG and no mannitol, even if flavouring is added. In particular, it has been found that a tablet comprising PEG and mannitol has a much lower requirement for lubricant or lubrication during tablet manufacture than a tablet comprising PEG but no mannitol. A high level of a lubricant in a tablet generally makes the tablet have an unacceptable taste. The reduced level (or absence of) a lubricant as compared with a tablet comprising PEG but no mannitol brings about an improved palatability (taste and mouthfeel) of a chewable or suckable tablet comprising PEG and mannitol.

Typically, solid formulations of dry ingredients are manufactured using dry granulation followed by punching with punch and die equipment. In a punch and die machine, dry ingredients are compressed together. It has surprisingly been found that a solid formulation of the invention comprising PEG and mannitol in the specified proportions has better structural integrity and is more convenient to manufacture than a solid formulation comprising PEG and no mannitol, or a smaller proportion of mannitol. Solid formulations of the invention are less susceptible to capping and laminating during punch and die manufacture than solid formulations comprising a smaller proportion of mannitol, or no mannitol. Solid formulations that become capped or laminated during die pressing are not suitable for use and they become waste. It has been found by the current inventors that a solid formulation containing between 50 and 90% w/w PEG and 10 to 40% w/w mannitol has better tablet pressing characteristics than a solid formulation containing no mannitol or 10%w/w or less mannitol, for example less than 10% mannitol.

Mannitol makes up 10 to 40 % w/w of the solid formulation of the invention. In a preferred embodiment, mannitol makes up 10 to 30% w/w of the solid formulation. For example, mannitol makes up 10 to 25% w/w, for example 10 to 20% w/w, preferably 12 to 20 % w/w, more preferably 12 to 19% w/w, 12 to 18% w/w, or 12 to 17 % w/w. For example, mannitol may make up 14 to 20% w/w, 14 to 19% w/w or 14 to 18% w/w 14 to 17% w/w of the solid formulation of the invention. Mannitol may be provided in various physical forms. For example, mannitol is available commercially in granular, powder or spray-dried form. In a preferred embodiment, the mannitol is granular. Mannitol is commercially available from several suppliers, including Merck, SPI Polyols Inc and Roquette.

In an embodiment, the PEG and mannitol are present in a weight ratio of PEG:mannitol of 1.25:1 to 9:1 (e.g. 3:1 to 9:1, or 4:1 to 9:1), preferably 2:1 to 7:1, preferably 4:1 to 6:1 or 4:1 to 8:1, for example 5:1 to 6:1. In preferred embodiments the ratio of PEG to mannitol 5:1 or thereabout.

The structural integrity of the solid formulation is retained when the mannitol is granular mannitol. It is surprising that the solid formulation of the invention is so structurally sound with granular mannitol. In general it is found that granular mannitol cannot be used with concentrations of other materials exceeding 25% by weight (Handbook of Pharmaceutical Excipients, 5th Ed., Pharmaceutical Press, 2006, page 452). The current inventors have found that the solid formulations of the invention, which comprise 60 to 90% w/w of materials other than mannitol, are readily manufacturable and have good structural integrity.

It has also surprisingly been found that a solid formulation of the invention comprising PEG and mannitol in the specified proportions is less prone to sticking to punch and die equipment than a solid formulation comprising PEG and a smaller proportion of mannitol, or no mannitol. This is particularly important when manufacturing formulations of the present invention at commercial scale since fouling of the manufacturing machinery may lead to manufacturing down-time with the increased costs associated therewith.

Lubricants can be included in tablet formulations to reduce the propensity for them to stick to the punch or die after die pressing. Examples of lubricants include magnesium stearate, potassium stearate, talc, stearic acid, sodium lauryl sulphate, and paraffin. Mixtures of different lubricants may be used. It has been found that a solid formulation of the invention comprising PEG and mannitol in the specified proportions requires a smaller proportion of lubricant to satisfactorily avoid sticking than a tablet comprising PEG and a smaller proportion of mannitol, or no mannitol. Preferably, a solid formulation of the invention comprises lubricant in an amount of 2.0% w/w or less, for example 1.5% w/w or less, or 1.0% w/w or less. For example it may comprise lubricant in an amount of 0.1 to 0.9% w/w, for example 0.2 to 0.8% w/w, preferably 0.3 to 0.7% w/w. For example, the lubricant is present in ratio of solid of component (b) (such as mannitol): lubricant ratio of 170:1 to 16:1, for example 57:1 to 20:1. A particularly preferred lubricant is magnesium stearate. If the lubricant is magnesium stearate, it is effective to satisfactorily avoid sticking when used at a level of under 1% w/w. Accordingly, in an embodiment, the tablet of the invention further comprises magnesium stearate in an amount of 0.1 to 0.9% w/w, for example 0.2 to 0.8% w/w, preferably 0.3 to 0.7% w/w, more preferably 0.5% w/w. It is surprising that magnesium stearate is effective at these levels as, in general, magnesium stearate is required at a level of over 1% in formulations comprising mannitol (Handbook of Pharmaceutical Excipients, 5th Ed., Pharmaceutical Press, 2006, page 452).

Thus the present invention provides a solid formulation for administration as a solid comprising;
(a) 50 - 90 % w/w polyethylene glycol (PEG) having an average molecular weight within the range 2,000 to 10,000 Da;
(b) 10 - 40 % w/w of mannitol; and
(c) 0.1 to 0.9% (e.g. 0.2 to 0.8%, 0.3 to 0.7% such as 0.5%) w/w of a lubricant such as magnesium stearate.

In some embodiments, the ratio of mannitol:lubricant is preferably 10:1 or greater, preferably, 20:1 or greater e.g. 25:1 or greater such as 30:1 or greater (e.g. 30:1 to 35:1 such as 30.6:1 or 32.4:1).

In an embodiment, a solid formulation of the invention does not include any added flavouring. In a preferred embodiment, a solid formulation of the invention includes at least one flavouring. Suitable flavourings are available from various flavour manufacturers and suppliers, for example International Flavours and Fragrances Inc. (Duddery Hill, Haverhill, Suffolk, CB9 8LG, United Kingdom), Ungerer & Company (Sealand Road, Chester, CH1 4LP, United Kingdom), Firmenich (Firmenich UK Ltd., Hayes Road, Southall, Middlesex, UB2 5NN, United Kingdom) or S. Black Ltd (Foxholes Business Park, John Tate Road, Hertford, Herts, SG13 7YH, United Kingdom). Examples of suitable flavours include orange, lemon-lime, lemon, citrus, chocolate, tropical fruit, aloe vera, peppermint, tea, strawberry, grapefruit, blackcurrant, pineapple and vanilla, raspberry-lemon, cola flavour, and combinations thereof.

Preferred flavours are peppermint and raspberry-lemon flavour.

A flavouring may be integral in a solid formulation, or it may be coated onto its surface. In one embodiment, the flavouring is integral in the solid formulation. In such a solid formulation, the flavouring preferably makes up 0.1 to 15% w/w of the solid formulation. For example, the flavouring may make up 0.1 to 5% w/w of the solid formulation, for example 0.1 to 2.0% w/w, for example 0.2 to 2.0% w/w. When the flavouring is peppermint, it is preferably present at a level of 0.1 to 1.0% w/w, for example 0.15 to 0.5% w/w. This level is particularly preferred when the solid of component (b) such as mannitol is present at a level of 14 to 17 % w/w of the solid formulation of the invention. When the flavouring is raspberry-lemon, it is preferably present at a level of 0.5 to 2.0% w/w, for example 1.0 to 2.0%, for example 1.2 to 1.8% w/w. This level is particularly preferred when the solid of component (b) such as mannitol is present at a level of 14 to 17% w/w of the solid formulation of the invention.

In an embodiment, the solid of component (b) such as mannitol and flavouring are, for example, present in a ratio of solid : flavouring of 170:1 to 3:1; when the flavouring is peppermint, the solid of component (b) such as mannitol and flavouring are preferably present in a ratio of solid : flavouring of 113:1 to 28:1. When the flavouring is raspberry-lemon, the solid of component (b) such as mannitol and flavouring are preferably present in a ratio of solid of component (b): flavouring of 14:1 to 7:1.

A solid formulation of the invention may comprise one or more sweeteners. Sweeteners may be sugar-based. Preferably, they are not sugar-based. Preferred sweeteners include aspartame, acesulfame potassium (acesulfame K), sucralose and saccharine or combinations thereof. Alternatively, it can be preferred for formulations of the invention to be substantially free from added sweeteners, for example to minimize the number of different components in the formulations. When present, sweeteners may, for example, be present in an amount of 0.01 to 1 % w/w. More preferably, a sweetener may be present in an amount of 0.1 to 1% w/w. The level of sweetener required to obtain a satisfactory taste may depend on the presence, and identity and quantity, of the other components of the formulation.

In general it is not necessary for a solid formulation of the invention to include preservatives or anti-oxidants. Nevertheless, low levels of anti-oxidants or preservatives may be included if required. It is preferred that formulations of the present invention are also substantially free from "salt taste" masking agents, such as agents that mask the taste of sodium sulphate, (other than flavourings mentioned herein) and from salts of non-fatty acids such as salts of mineral acids.

A solid formulation of the invention can be of any convenient size. As mentioned above, a tablet should be sufficiently large to provide the desired quantity of PEG to the subject, but not be so large as to be uncomfortable in the mouth, difficult to chew or suck, or difficult to package. A tablet may, for example have a mass of 0.5 to 10g, more preferably 0.5 to 5g, for example 1.0 to 5.0g, for example 2.0 to 3.5g, for example 2.5 to 3.5g. In one embodiment, a tablet of the invention has a mass of from 2.5 to 3.0g, for example 2.75g. For certain uses, where a larger amount of PEG is to be delivered to the subject, a larger tablet may be convenient, for example having a mass of 3 to 10g, for example 3 to 5g, 3 to 7g, 4 to 7g, or 5 to 8g, for example 4 to 7g. For certain uses, where a smaller amount of PEG is to be delivered to the subject, for example for paediatric uses, a smaller tablet may be convenient, for example having a mass of 0.5 to 2.0g, for example 1.0 to 1.75g, for example 1.25 to 1.50g.

A solid formulation of the invention may therefore be a solid formulation of mass 2.0 to 3.5g comprising:
(a) 1.00 - 3.15g polyethylene glycol (PEG) having an average molecular weight within the range 2,000 to 10,000 Da; and
(b) 0.20 - 1.40g of the solid such as mannitol.

A solid formulation of the invention may therefore be a solid formulation of mass 2.5 to 3.5g comprising:
(a) 1.25 - 3.15g polyethylene glycol (PEG) having an average molecular weight within the range 2,000 to 10,000 Da; and
(b) 0.25 - 1.40g of the solid such as mannitol.

Similarly, a formulation of the invention may therefore be a solid formulation of mass 1.0 to 1.75g comprising:
(a) 0.50 - 1.575g polyethylene glycol (PEG) having an average molecular weight within the range 2,000 to 10,000 Da; and
(b) 0.10 - 0.70g of the solid such as mannitol;

As mentioned above, a lubricant (for example magnesium stearate) may be present in a solid formulation of the invention in an amount of 2% w/w or less, for example 1% w/w or less. A solid formulation of the invention of mass 2.0 to 3.5g may therefore comprise 0.07g or less of lubricant, for example 0.35g or less of lubricant. For example, it may comprise lubricant in an amount of 0.002 to 0.0315g, for example 0.004 to 0.028g, for example 0.006 to 0.0245g. A larger formulation of the invention of mass 3.0 to 7.0g may comprise 0.14g or less of lubricant, for example 0.07g or less of lubricant. For example, it may comprise lubricant in an amount of 0.003 to 0.063g, for example 0.006 to 0.056g, for example 0.009 to 0.049g. A smaller formulation of the invention of mass 1.0 to 1.75g may comprise 0.035g or less of lubricant, for example 0.0175g or less of lubricant. For example, it may comprise lubricant in an amount of 0.001 to 0.01575g, for example 0.002 to 0.014g, for example 0.003 to 0.01225g.

As mentioned above, flavouring may be present in a solid formulation of the invention and, when present, it preferably makes up 0.1 to 15% w/w of the solid formulation. A solid formulation of the invention of mass 2.0 to 3.5g may therefore comprise 0.002 to 0.525g of flavouring, for example 0.002 to 0.175g, for example 0.002 to 0.07g, for example 0.004 to 0.07g of flavouring. A larger formulation of the invention of mass 3.0 to 7.0g may comprise 0.003 to 1.05g of flavouring, for example 0.003 to 0.35g, for example 0.003 to 0.14g, for example 0.006 to 0.14g of flavouring. A smaller formulation of the invention of mass 1.0 to 1.75g may comprise 0.001 to 0.2625g of flavouring, for example 0.001 to 0.0525g for example 0.001 to 0.021g, for example 0.002 to 0.021g of flavouring.

For example, a solid formulation of the invention may comprise:
(a) 50 - 90 % w/w polyethylene glycol (PEG) having an average molecular weight within the range 2,000 to 10,000 Da;
(b) 10 - 30 % w/w of the solid such as mannitol;
(c) 0.1 - 2.0 % w/w lubricant; and
(d) 0.1 - 15 % w/w flavouring.

In one embodiment, a solid formulation of the invention comprises:
(a) 70 - 90 % w/w polyethylene glycol (PEG) having an average molecular weight within the range 3,000 to 8,000 Da;
(b) 10 - 25 % w/w of the solid such as mannitol;
(c) 0.1 - 1.5 % w/w magnesium stearate; and
(d) 0.1 - 2.0 % w/w flavouring.

For example, a solid formulation of the invention comprises:
(a) 75 - 89 % w/w polyethylene glycol (PEG) having an average molecular weight within the range 3,000 to 4,000 Da;
(b) 10 - 20 % w/w of the solid such as mannitol;
(c) 0.2 - 0.8 % w/w magnesium stearate; and
(d) 0.1 - 1.0 % w/w flavouring.

For example, a solid formulation of the invention may comprise:
(a) 1.00 - 3.15g polyethylene glycol (PEG) having an average molecular weight within the range 2,000 to 10,000 Da;
(b) 0.20 - 1.40g of the solid such as mannitol;
(c) 0.002 - 0.07g lubricant; and
(d) 0.002 - 0.525g flavouring.

In one embodiment, a solid formulation of the invention comprises:
(a) 1.40 - 3.15g polyethylene glycol (PEG) having an average molecular weight within the range 3,000 to 8,000 Da;
(b) 0.20 - 0.875g of the solid such as mannitol;
(c) 0.002 - 0.0525g magnesium stearate; and
(d) 0.002 - 0.07g flavouring.

For example, a solid formulation of the invention comprises:
(a) 1.5 - 3.115g polyethylene glycol (PEG) having an average molecular weight within the range 3,000 to 4,000 Da;
(b) 0.20 - 0.70g of the solid such as mannitol;
(c) 0.004 - 0.016g magnesium stearate; and
(d) 0.002 - 0.035g flavouring.

For example, a solid formulation of the invention comprises:
(a) 2273 to 2284mg polyethylene glycol (PEG) having an average molecular weight within the range 3,000 to 4,000 Da;
(b) 420 to 446mg of the solid such as mannitol;
(c) 13.5 to 13.75mg magnesium stearate; and
(d) 11 to 42mg of flavouring such as peppermint or raspberry/lemon flavouring.

The solid formulations of the invention may be packaged in any convenient fashion. For example a plurality of units (e.g. tablets) of the solid formulation of the present invention (such as 5, 10, 15 or 20) may be packaged in a way conventional in the vitamin supplements industry. For example, they may be packed in a tube (such as a PTFE tube) equipped with a removable and replaceable closing means, for example a stopper. Alternatively, the solid formulations of the invention may be provided in a jar or other container with a removable and replaceable lid, or in a bag or within a wrapper (for example a foil wrapper). A desiccant is preferably also present. Alternatively, they may be packaged in a blister pack. In an embodiment, the solid formulations are packaged within a tube, jar, bag, wrapper or other container without any wrapping around individual units (e.g. tablets). Optionally, individual units of solid formulations of the present invention may have a wrapping. In a preferred embodiment, 30 units of the formulation of the present inventions are provided spilt into three tubes (e.g. 10 units per tube) or other packaging optionally together with instructions for use. Units of the present invention may also be provided in a refill bag enabling previously obtained tubes to be refilled with units of the invention.

The solid formulations of the invention can be taken on their own as presented and chewed or sucked by a subject. It is not necessary for a subject to take water or another drink with the solid formulation. Some subjects may wish to drink water or another fluid with or soon after taking a solid formulation of the invention so as to facilitate the intake. The convenient packaging and the lack of a need to take water or another drink greatly increases the convenience of the solid formulations to subjects in comparison with other forms of PEG-based products currently on the market. Formulations of the present invention may be consumed prior to eating a meal or snack, together with the meal or snack or following a meal or snack.

In one embodiment, a solid formulation of the invention may be used to prevent gastrointestinal disorders. The formulation may be particularly beneficial for maintenance of good health, in particular maintenance of good gastrointestinal health. It may, for example, prevent dehydration of the stool, soften the stool for ease of defaecation, prevent constipation and allow regular gastrointestinal transit. The solid formulation of the invention may be used to promote stool softening, increase stool weight and/or increase stool frequency in healthy subjects. Improvement of those features may lead to an increased sensation of well-being.

Also disclosed herein is a method of preventing gastrointestinal disorders in a healthy subject, for example softening the stool, increasing stool weight and/or increasing stool frequency, preventing dehydration of the stool, softening the stool for ease of defaecation or preventing constipation in a healthy subject, comprising administering a solid formulation according to the invention. In particular, disclosed herein is a method of non-therapeutically preventing gastrointestinal disorders in a healthy subject, for example softening the stool, increasing stool weight and/or increasing stool frequency, preventing dehydration of the stool, softening the stool for ease of defaecation or preventing constipation in a healthy subject.

As used herein the term "non-therapeutic" and grammatical variations thereof means that the formulations of the present invention preferably do not have a measurable pharmacological, immunological or metabolic effect on the body.

Formulations of the present invention may be particularly suited for subjects of more than 50 years of age, for example more than 60, 65 or 70 years of age. Healthy subjects of the present invention may be those who are particularly susceptible to episodes of constipation and wish to delay or reduce the frequency of such episodes or prevent the onset of constipation. By doing so, formulations of the present invention may reduce the need for such subjects to seek more interventional measures to treat constipation such as the use of laxatives. Formulations of the invention may also prevent straining at stool which may be particularly beneficial for subjects who are healthy (i.e. have normal bowel movement) but are hypertensive where such straining carries a degree of risk to the subject.

Subjects of the present invention are mammals and typically human.

In one embodiment, the subject typically takes up to 6g (or thereabout) per day of PEG, for example 2 to 6g per day, for example 3 to 5g per day, for example 4 to 5g per day. In that embodiment, the formulation is free from components of a nature and quantity having a laxative effect. PEG is not considered to have significant laxative activity in an adult when taken at a level of 6g per day. Mannitol, flavouring and lubricant components are also not considered to have significant laxative activity at the daily levels at which they are provided when the formulation provides up to 6g PEG per day. For a solid formulation of 2.0 to 3.5g total mass and comprising 85% PEG w/w, a healthy subject may be recommended to take 1 or 2, or 1, 2 or 3 per day (to provide up to 6g or thereabout of PEG per day). For a smaller solid formulation (of, for example, 1.0 to 1.75g total mass), a healthy subject may be recommended to take 1 to 6 per day, for example 2 to 5 per day (to provide up to 6g or thereabout of PEG per day). Conversely, for a larger solid formulation (of, for example, total 3.0 to 7.0g total mass), a healthy subject may be recommended to take 1 or 2 per day (to provide up to 6g or thereabout of PEG per day).

Formulations of the present invention are particularly suited in enabling the subject to consume up to 6g (or thereabout) of PEG on a chronic basis. Preferably, the formulations of present invention enable a healthy subject to consume 4 to 5 g (for example 4g or thereabout) of PEG per day Such an amount of PEG, taken on a chronic (e.g. daily) basis, has the advantages mentioned *supra* in terms of preventing gastrointestinal disorders, (particularly those related to defecation), and maintaining gastrointestinal health without causing excessive gastrointestinal disturbances (for example loose stools).

While consuming a higher amount of PEG (for example 10g or more) on a chronic basis may be effective in preventing or indeed treating disorders of the gastrointestinal system, such an amount is likely to produce undesirable gastrointestinal disturbances. As mentioned *supra*, if the subject consuming such higher amounts of PEG are otherwise healthy, they may be more likely to discontinue consumption regardless of any benefit in continuing, if such consumption is uncomfortable, inconvenient or otherwise unpleasant.

Therefore, the present invention provides a solid formulation for use in preventing gastrointestinal disorders or maintaining gastrointestinal health as described *supra* in a healthy subject which formulation is of a size that is not too large so as to be uncomfortable to consume, has good mouthfeel and ease of manufacture yet provides an effective amount of PEG without the need to consume so many units of the formulation so as to be bothersome. In doing so, the formulation of the present invention enables chronic use on a daily or other regular basis by a healthy subject.

Thus, the present invention provides a solid formulation for oral administration as a solid (preferably having a mass of 1.0 to 5.0g) to a healthy subject (such as a human) for use in a method of preventing gastrointestinal disorders or maintaining gastrointestinal health, for example for softening the stool, increasing stool weight and/or increasing stool frequency, preventing dehydration of the stool, softening the stool for ease of defaecation or preventing constipation,
wherein the formulation comprises:
(a) 50 - 90 % w/w (for example 60 to 90% w/w, preferably 70 to 90% w/w, more preferably 70 to 89 % w/w, for example 75 to 89 % w/w, e.g. 78 to 89 % w/w, 80 to 85% w/w, 81 to 85 % w/w, 80 to 84 % w/w, 82 to 84 % w/w) polyethylene glycol (PEG) having an average molecular weight within the range 2,000 to 10,000 Da; and
(b) 10 - 40 % w/w (for example 10 to 25% w/w, 10 to 20% w/w, preferably 12 to 20 % w/w, more preferably 12 to 19% w/w, 12 to 18% w/w, or 12 to 17 % w/w, e.g.14 to 20% w/w, 14 to 19% w/w or 14 to 18% w/w 14 to 17% w/w) of the solid such as mannitol; together with
(c) optional lubricant, optional flavouring and/or optional sweetener as described supra; and
wherein the method comprises administering said formulation so that the subject consumes up to 6g (or thereabout) of PEG per day.

Preferably, said method comprises administering said formulation so that the subject consumes between 2g and 6g, e.g. 2g to 5.5g per day. Preferably, said method is performed on a daily or alternate day basis. Preferably the method is performed over a period of at least two weeks, preferably at least a calendar month, more preferably at least 6 consecutive calendar months, or at least 12 consecutive calendar months, or at least 24 or at least 36 consecutive calendar months.

The present invention also provides a tablet comprising 2g of PEG 3350 or thereabout and raspberry-lemon or mint flavour for preventing gastrointestinal disorders, preventing dehydration of the stool, softening the stool for ease of defaecation, preventing constipation and allowing regular gastrointestinal transit. The subject may consume 1 to 2 tablets daily. The tablet may be part of a multi-tablet pack of e.g. thirty tablets. A multi-tablet pack, eg a cardboard box, may be further divided into or may itself contain containers each comprising e.g. ten tablets; for example, a cardboard box may contain three tubes, each containing ten tablets. The container(s), such as the tubes, preferably have tamper-evident seals. The pack and/or container(s) preferably further contain(s) instructions for use of the formulations. The instructions for use preferably instruct the subject to place individual tablets in the mouth and either chew or suck until they have fully dissolved; optionally, a glass of water can be drunk to help in the method.

PEG has also been described as being effective in preventing cancer of the colon and/or rectum (see EP1124566). Accordingly, in a further embodiment, a solid formulation of the invention may be used as a PEG-based composition treat or prevent cancer of the colon and/or rectum (for example colorectal cancer). A method of treating or preventing cancer of the colon and/or rectum in a subject comprising administering to the subject a solid formulation of the invention is also provided. Suitable daily amounts of PEG to be effective in this embodiment are for example 6.5g or more, for example 8.0g or more, for example 6.5 to 100g, for example 8 to 50g PEG per day. To achieve the desired amount of PEG, a subject may be administered a suitable number of solid formulations of the invention of suitable PEG content. The number of solid formulations required depends on the concentration of PEG in the formulations and the mass of each solid formulation. A reduction in the total amount of PEG in each solid formulation brings about a pro rata increase in the number of solid formulations that a subject will need to take.

In this embodiment, solid formulations of the invention are preferably substantially free from components of a nature and quantity having a laxative effect, other than PEG and/or mannitol.

It will be apparent to the reader of this specification, that the term "comprising" and grammatical variations thereof, in relation to embodiments of the invention described *supra,* may instead be "consisting essentially of" or "consisting of'.

### EXAMPLES

### Example 1: Comparison of PEG tablets without mannitol with PEG tablets including mannitol

The tablets described in Table 1a were prepared. The materials were dispensed and then bag blended. The unit formula amounts were compressed on a Manesty D machine at normal manufacturing speed and with a standard stainless steel punch and die with flat 22mm diameter and beveled edge and PTFE inserts. The properties of the tablets were noted and they are given in Table 1b below. In the tables, * denotes comparative examples.

It is seen in Tables 1a and 1b that tablet 1A, made up only of PEG, stuck to the punches and were capped and chipped. It was possible to reduce the capping and chipping to an extent by including magnesium stearate, as seen in tablets 1B, 1C and 1D. However, tablets including magnesium stearate had a poor taste, and the hardness was poor, particularly with the higher amounts of magnesium stearate in tablets 1C and 1D. In contrast, tablet 1E that includes mannitol had a better taste, good hardness and was well manufactured with only minimal capping.

It is seen that addition of mannitol provides a tablet that has good processing characteristics with only 0.5% w/w of magnesium stearate lubricant. It is also seen that a pleasant taste is achieved in the tablet containing mannitol, something not achieved in the tablets lacking mannitol.

**Table 1a - Composition of Tablets 1A to 1E**

| **Component** | **Tablet 1A*** | | **Tablet 1B*** | | **Tablet 1C*** | | **Tablet 1D*** | | **Tablet 1E** | |
|---|---|---|---|---|---|---|---|---|---|---|
| | Unit formula | 5kg bag blend | Unit formula | 5kg bag blend | Unit formula | 5kg bag blend | Unit formula | 5kg bag blend | Unit formula | 5kg bag blend |
| **PEG average MW 3000-4000** | 3000mg (100%) | 5.00kg | 2985mg (99.5%) | 4.98kg | 2700mg (90%) | 4.50kg | 2400mg (80%) | 4.00kg | 2535mg | 4.23kg |
| **Mannitol** | - | - | - | - | - | - | - | - | 450mg (15%) | 0.75kg |
| **Magnesium Stearate** | - | - | 15mg (0.5%) | 0.03kg | 300mg (10.0%) | 0.5kg | 600mg (20%) | 1.00kg | 15mg (0.5%) | 0.03kg |
| **Total wt** | 3000mg | | 3000mg | | 3000mg | | 3000mg | | 3000mg | |

**Table 1b - Properties of Tablets 1A to 1E**

| **Ease of Manufacture** | Capping, chipping and sticking to punches | Poor hardness and weight. Difficult to compress. | Some capping. Difficult to compress. | Unable to form tablet | Slight capping |
|---|---|---|---|---|---|
| **Taste** | Poor taste | Poor taste | Poor taste | | Better taste |
| **Hardness** | 6.37-13.59kg | 2.47-5.60kg | 0.7-1.8kg | | 0.4-6.1kg |

### Example 2: Comparison of tablets containing different w/w % of mannitol

The tablets described in Table 2 were prepared by combining the dry ingredients and compressing in a punch and die machine. For tablets 2A to 2C, the machine was a Manesty 16 punch D machine with a standard stainless steel punch and die with flat 22mm diameter and beveled edge with PTFE and vulcalon inserts from I Holland Ltd. For tablets 2D and 2E, the unit formula amounts were compressed on a Manesty D machine at normal manufacturing speed and with a standard stainless steel punch and die with flat 22mm diameter and beveled edge. The properties of the tablets were noted and they are given in Table 2b below. In the tables, * denotes comparative example.

Tablet 2A had an acceptable taste. However, the tablets were prone to sticking to the tableting machine, and many tablets were capped or laminated, making them unusable. Tablet 2B contained the same flavouring as tablet 2A, but more mannitol (15.3% vs. 9.1% in 2A) and less flavouring (1.5% vs. 5.4%). Tablet 2B had an acceptable taste and there was no evidence of sticking to the tableting machine, or capping or laminating of the tablets. Tablet 2C contains similar amounts of PEG, mannitol and magnesium stearate to tablet 2B, but the flavouring is peppermint. It displays similar characteristics to tablet 2B. Tablet 2D contained no flavouring, and 10% mannitol. It displayed no capping or sticking and only a small amount of chipping. Tablet 2E contained no flavouring, and 40% mannitol. It displayed good manufacturing characteristics. Tablets 2D and 2E had a bland taste as compared with tables 2A to 2C. This is most likely because of the absence of flavouring. The taste was, however, not unpleasant. Tablets 2F to 2H all displayed good manufacturing characteristics and an acceptable taste.

It is seen that a tablet containing from 59.5 to 89.5% w/w PEG (in particular 82.7 or 82.9% w/w PEG) and 10 to 40% /w mannitol (in particular 15.3 or 16.2% w/w mannitol) has better ease of manufacture characteristics than a tablet containing 85.0% PEG and 9.1% mannitol.

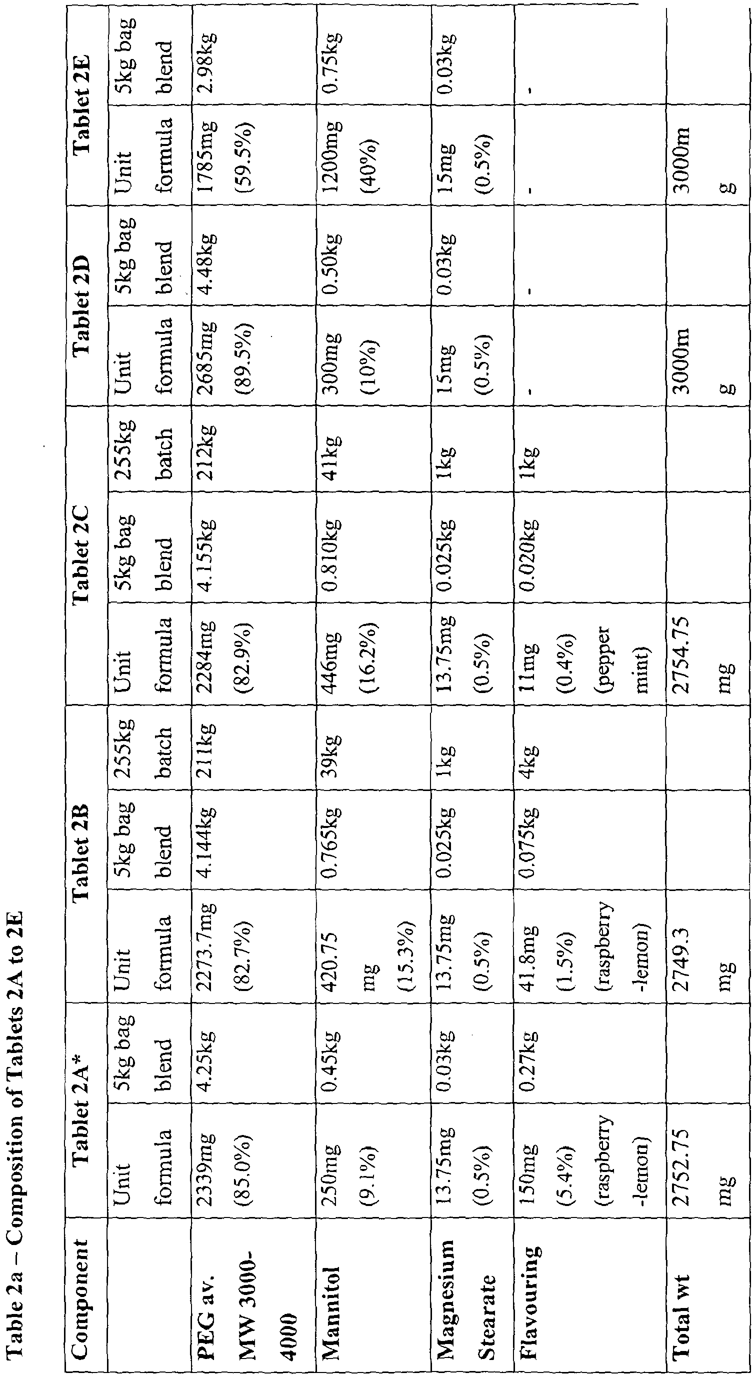

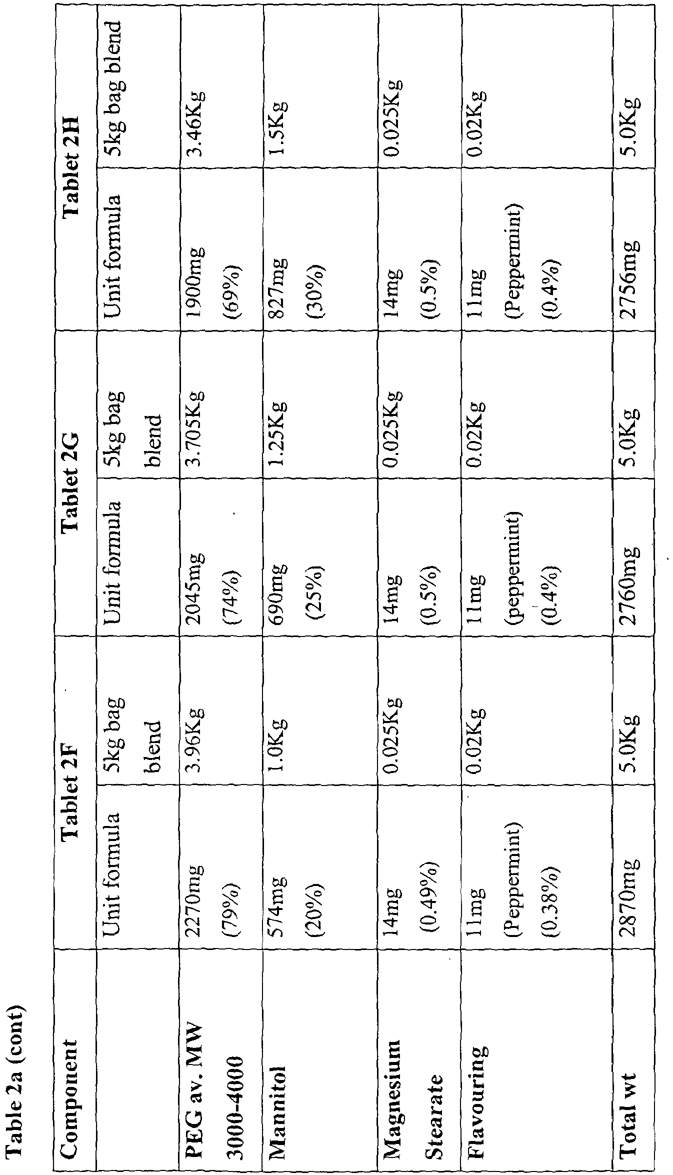

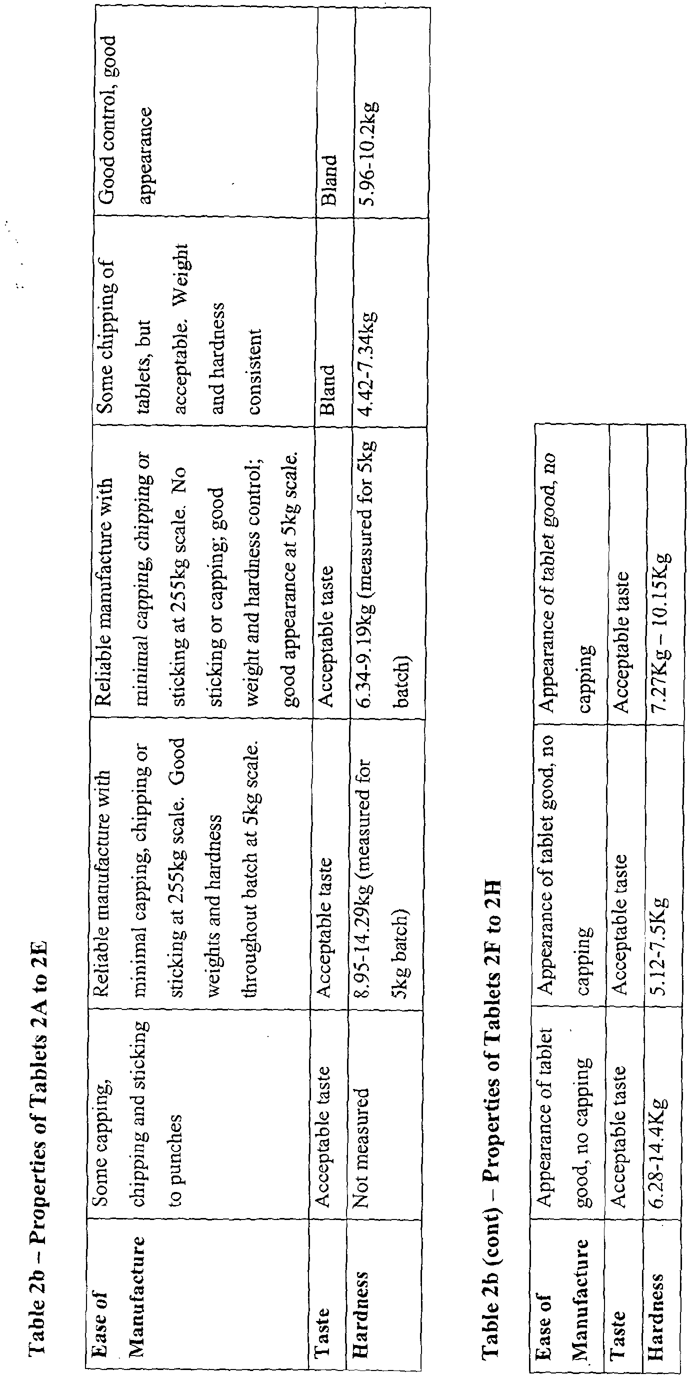

### Example 3: Comparison of tablets containing mannitol and different w/w % of magnesium stearate

The tablets described in Table 3a were prepared. The materials were dispensed and then bag blended. The unit formula amounts were compressed on a Manesty D machine at normal manufacturing speed and with a standard stainless steel punch and die with flat 22mm diameter and beveled edge and PTFE inserts. The properties of the tablets were noted and they are given in Table 3b below. In the tables, * denotes comparative example.

It is seen in Tables 3a and 3b that tablets containing PEG and 15% w/w mannitol and 0.2%, 0.5% or 5.0% w/w magnesium stearate have good manufacturing properties and acceptable taste.

**Table 3a - Composition of Tablets 3A to 3C**

| **Component** | **Tablet 3A** | | **Tablet 3B** | | **Tablet 3C** | |
|---|---|---|---|---|---|---|
| | Unit formula | 5kg bag blend | Unit formula | 5kg bag blend | Unit formula | 5kg bag blend |
| PEG average MW 3000-4000 | 2544mg (84.8%) | 4.24kg | 2400mg (80%) | 4.0kg | 2535mg (84.5%) | 4.23kg |
| Mannitol | 450mg (15%) | 0.75kg | 450 mg (15%) | 0.75kg | 450mg (15%) | 0.75kg |
| Magnesium Stearate | 6mg (0.2%) | 0.01kg | 150mg (5%) | 0.25kg | 15mg (0.5%) | 0.03kg |
| Flavouring | - | - | - | - | - | - |
| Total wt | 3000 mg | | 3000 mg | | 3000 mg | |

**Table 3b - Properties of Tablets 3A to 3C**

| **Ease of Manufacture** | Tablets good, no capping | Tablets good | Slight capping |
|---|---|---|---|
| **Taste** | Bland taste | Bland taste, slightly artificial | Better taste |
| **Hardness** | 5.4-11.0kg | 4.42-7.34kg | 0.4-6.1kg |

### Example 4: Comparison of various flavours of PEG+mannitot tablets

Tablets analogous to the tablets of Example 2 with a variety of flavourings were prepared by combining the dry ingredients and compressing in a punch and die machine. The machine was a Manesty 16 punch D machine with a standard stainless steel punch and die with flat 22mm diameter and beveled edge with PTFE and vulcalon inserts from I Holland Ltd.

The tablets were provided to a panel of tasters. They were asked to taste each of the tablets and score them with an emphasis on the flavour, scoring from 1 (unpleasant) to 5 (pleasant). There were 22 tasters and their scores were summed together. It was found that peppermint flavoured tablets (score = 65) and lemon-raspberry flavoured tablets (score = 87) were preferred over lemon-lime flavoured tablets (score = 37) and orange flavoured tablets (score = 23).

### Example 5: Comparison of Tablets containing various other solids.

Tablets containing solids other than mannitol were prepared in the same manner as the tablets of Example 2 *supra.* The composition and properties of these tablets are noted in Table 5a and 5b below.

**Table 5a - Comparison of Alternative Solids**

| **Component** | **Tablet 5A** | **Tablet 5B** | **Tablet 5C** |
|---|---|---|---|
| | Unit amount/ 5Kg blend | Unit amount/ 5Kg blend | Unit amount/ 5Kg blend |
| **PEG av. MW 3000-4000** | 2276mg/4.145Kg (83%) | 2279mg/4.145Kg (83%) | 2280mg/4.145Kg |

| **Solid** | Sorbitol | Lactose/Starch¹ | Xylitol |
|---|---|---|---|
| | 445mg/0.81Kg (16.2%) | 446mg/0.81Kg (16.2%) | 446mg/0,81Kg |
| **Magnesium Stearate** | 14mg/0.025Kg (0.51%) | 14mg/0.025Kg (0.51%) | 14mg/0.025Kg (0.51%) |
| **Flavouring** | 11mg/0.02Kg (0.40%) | 11mg/0/02Kg (0.40%) | 11mg/0.02Kg (0.40%) |
| **Total wt** | 2746mg / 5.0Kg | 2753mg/5.0Kg | 2750mg/5.0Kg |

| | | | |
|---|---|---|---|
| 1. Lactose / starch compound, StarLac® (Roquette Pharma, Northants, UK) is a spray-dried compound consisting of 85% alpha-lactose monohydrate (Ph. Eur. /USP-NF) and 15% maize starch (Ph. Eur. /USP-NF) dry matter. | | | |

**Table 5b - Properties of Tablets 5A to 5C**

| **Ease of Manufacture** | Tablet appearance good, no capping | Tablet appearance good, no capping | Tablet appearance good, no capping |
|---|---|---|---|
| **Taste** | Tasted ok but lacked enhanced mouthfeel of mannitol tablets | Good hardness, tasted bland | Tasted ok, but tablet too soft |
| **Hardness** | 6.97-10.09Kg | 4.13-9.4Kg | 4.67-7.99Kg |

### Example 6 - Comparison of Tablets containing further various solids.

Tablets containing solids other than mannitol were prepared in the same manner as the tablets of example 2 *supra.* The composition and properties of these tablets are noted in Table 6a and 6b below.

**Table 6a - Comparison of alternative solids**

| **Component** | **Tablet 6A** | **Tablet 6B** | **Tablet 6C** |
|---|---|---|---|
| | Unit amount/5Kg blend | Unit amount/5Kg blend | Unit amount/5Kg blend |
| **PEG av.MW 3000-4000** | 2279mg/4.145Kg (82.9%) | 2279mg/4.145Kg (82.9%) | 2263mg/4.145Kg (82.9%) |
| **Solid** | Lactose | Dextrate¹ | Cellulose² |
| | 445mg/0.810Kg (16.2%) | 440mg/0.800Kg (16.0%) | 442mg/0.810Kg (16.2%) |
| **Magnesium Stearate** | 14mg/0.025Kg (0.5%) | 14mg/0.025Kg (0.50%) | 13.6mg/0.025Kg (0.5%) |
| **Flavouring** | 11mg/0.020Kg (0.4%) | 11mg/0.020Kg (0.4%) | 11mg/0.020Kg (0.4%) |
| **Total wt** | 2750mg/5.0Kg | 2746mg/4.99Kg | 2730mg/5.0Kg |

| | | | |
|---|---|---|---|
| ¹ Emdex®, available from JRS Pharma, Rosenberg, Germany. ² Avicel®, microcrystalline cellulose, available from FMC biopolymers, Philadelphia, USA. | | | |

**Table 6b - Properties of Tablets 6A to 6C**

| **Ease of Manufacture** | Tablet appearance good, no capping, hardness good | Tablet appearance good, some capping | Tablet appearance good, no capping |
|---|---|---|---|
| **Taste** | Taste ok, but very bland | Pleasant taste but quite soft | Taste unpleasant |
| **Hardness** | 5.2-13.6Kg | 5.4-11.9Kg | 7.1-13.4Kg |

## Claims

1. A solid formulation for oral administration as a solid, comprising:
(a) 50 - 90 % w/w polyethylene glycol (PEG) having an average molecular weight within the range 2,000 to 10,000 Da; and
(b) 10 - 40 % w/w of a solid selected from sorbitol, lactose, dextrates, cellulose, xylitol, maltitol, mannitol.

2. A solid formulation of claim 1 wherein the group further comprises lactose and starch (e.g. a compound comprising lactose monohydrate and maize starch such as Starlac®).

3. A solid formulation of any preceding claim comprising 82 to 84 % w/w PEG.

4. A solid formulation of any preceding claim comprising 10 to 20 % w/w of solid of component (b).

5. A solid formulation of any preceding claim wherein the solid of component (b) is mannitol, (for example granular mannitol), xylitol, lactose and starch, or sorbitol.

6. A solid formulation of claim 5 wherein the solid of component (b) is mannitol, for example granular mannitol.

7. A solid formulation of any preceding claim wherein the PEG has an average molecular weight of between 3,000 and 4,100 Da, for example within the range 3,000 to 4,000 Da.

8. A solid formulation of claim 1, comprising:
(a) 70 - 90 % w/w polyethylene glycol (PEG) having an average molecular weight within the range 2,000 to 10,000 Da;
(b) 10 - 20 % w/w of the solid such as mannitol;
(c) 0 - 2.0% w/w lubricant; and
(d) 0 - 2.0% w/w flavouring.

9. A solid formulation of any preceding claim, comprising PEG and mannitol in a weight ratio of PEG : mannitol = 3:1 to 9:1.

10. A solid formulation of any preceding claim comprising a lubricant in an amount of 2.0% w/w or less, for example 0.2 to 0.8% w/w, for example 0.5% w/w.

11. A solid formulation as claimed in claim 10 wherein the lubricant is magnesium stearate.

12. A solid formulation of any preceding claim comprising a flavouring.

13. A solid formulation of any preceding claim that is substantially free from electrolytes (e.g. sodium chloride, potassium chloride, bicarbonates such as sodium bicarbonate, sulphates such as sodium sulphate, or phosphates).

14. A solid formulation of any preceding claim that has a mass of 0.5 to 10g, for example 1.0 to 5.0g.

15. A solid formulation as claimed in claim 1 that has a mass of 2.0 to 3.5g and comprises:
(a) 1.00 - 3.15g polyethylene glycol (PEG) having an average molecular weight within the range 2,000 to 10,000;
(b) 0.20 - 1.40g mannitol.

16. A solid formulation as claimed in claim 1 that comprises;
(a) 2273 to 2284mg polyethylene glycol (PEG) having an average molecular weight within the range 3,000 to 4,000 Da;
(b) 420 to 446mg of solid such as mannitol;
(c) 13.5 to 13.75mg magnesium stearate; and
(d) 11 to 42mg flavouring, such as peppermint or raspberry/lemon flavouring.

17. A solid formulation of any preceding claim for use in the prevention of gastrointestinal disorders or maintaining gastrointestinal health for example, preventing dehydration of the stool, softening the stool for ease of defaecation, preventing constipation and allowing regular gastrointestinal transit in a subject (e.g. human).

18. A formulation of claim 17 wherein the subject is healthy (that is has normal bowel movement).

19. A formulation of claim 18, for use in delaying or reducing the frequency of episodes of constipation or preventing the onset of constipation.

20. A solid formulation as claimed in any one of claim 1 to 16 wherein the subject (e.g. human) consumes a sufficient amount of formulation to receive up to 6g (or thereabout) per day of PEG.

21. A chewable or suckable solid formulation for oral administration as a solid comprising;
(a) 2.0 to 3.5g PEG (for example 2.1 to 2.5g) having an average molecular weight of 3000 to 4000 Da;
(b) 250 to 500mg (for example 420 to 450mg) of a solid selected from sorbitol, lactose, dextrates, cellulose, xylitol, maltitol, mannitol;
(c) optional flavouring which if present is in an amount of 5 to 75mg;
(d) optional lubricant such as magnesium stearate which if present is in an amount of 5 to 25mg.

22. The formulation of claim 21 for use in preventing dehydration of the stool, softening the stool for ease of defecation, preventing constipation, allowing regular gastrointestinal transit.

23. A pack comprising a plurality of units of solid formulations according to any one of claims 1 to 16 and 20 to 22, for example 5 or more (such as 10), 10 or more (such as 20) or 20 or more (such as 30) units of the solid formulation.

## Patentansprüche

1. Eine feste Formulierung zur oralen Verabreichung als Feststoff, umfassend:
(a) 50 - 90 % Gew./Gew. Polyethylenglycol (PEG) mit einem mittleren Molekulargewicht im Bereich von 2000 bis 10000 Da, und
(b) 10 - 40 % Gew./Gew. eines Feststoffs, ausgewählt aus Sorbit, Lactose, Dextraten, Cellulose, Xylit, Maltit, Mannit.

2. Eine feste Formulierung nach Anspruch 1, wobei die Gruppe ferner Lactose und Stärke umfasst (z.B. eine Verbindung, die Lactose-Monohydrat und Maisstärke umfasst, wie z.B. Starlac®).

3. Eine feste Formulierung nach einem vorhergehenden Anspruch, umfassend 82 bis 84 % Gew./Gew. PEG.

4. Eine feste Formulierung nach einem vorhergehenden Anspruch, umfassend 10 bis 20 % Gew./Gew. Feststoff der Komponente (b).

5. Eine feste Formulierung nach einem vorhergehenden Anspruch, wobei der Feststoff der Komponente (b) Mannit (zum Beispiel granulatförmiger Mannit), Xylit, Lactose und Stärke, oder Sorbit ist.

6. Eine feste Formulierung nach Anspruch 5, wobei der Feststoff der Komponente (b) Mannit ist, zum Beispiel granulatförmiger Mannit.

7. Eine feste Formulierung nach einem vorhergehenden Anspruch, wobei das PEG ein mittleres Molekulargewicht zwischen 3000 und 4100 Da besitzt, zum Beispiel im Bereich von 3000 bis 4000 Da.

8. Eine feste Formulierung nach Anspruch 1, umfassend:
(a) 70 - 90 % Gew./Gew. Polyethylenglycol (PEG) mit einem mittleren Molekulargewicht im Bereich von 2000 bis 10000 Da,
(b) 10 - 20 % Gew./Gew. des Feststoffs wie z.B. Mannit,
(c) 0 - 2,0 % Gew./Gew. Gleitmittel und
(d) 0 - 2,0 % Gew./Gew. Aromastoff.

9. Eine feste Formulierung nach einem vorhergehenden Anspruch, umfassend PEG und Mannit in einem Gewichtsverhältnis von PEG : Mannit von 3:1 bis 9:1.

10. Eine feste Formulierung nach einem vorhergehenden Anspruch, umfassend ein Gleitmittel in einer Menge von 2,0 % Gew./Gew. oder weniger, zum Beispiel 0,2 bis 0,8 % Gew./Gew., zum Beispiel 0,5 % Gew./Gew.

11. Eine feste Formulierung wie in Anspruch 10 beansprucht, wobei das Gleitmittel Magnesiumstearat ist.

12. Eine feste Formulierung nach einem vorhergehenden Anspruch, umfassend einen Aromastoff.

13. Eine feste Formulierung nach einem vorhergehenden Anspruch, die im Wesentlichen frei von Elektrolyten (z.B. Natriumchlorid, Kaliumchlorid, Bicarbonaten wie z.B. Natriumbicarbonat, Sulfaten wie z.B. Natriumsulfat, oder Phosphaten) ist.

14. Eine feste Formulierung nach einem vorhergehenden Anspruch, die ein Gewicht von 0,5 bis 10 g besitzt, zum Beispiel 1,0 bis 5,0 g.

15. Eine feste Formulierung wie in Anspruch 1 beansprucht, die ein Gewicht von 2,0 bis 3,5 g besitzt und umfasst:
(a) 1,00 - 3,15 g Polyethylenglycol (PEG) mit einem mittleren Molekulargewicht im Bereich von 2000 bis 10000,
(b) 0,20 - 1,40 g Mannit.

16. Eine feste Formulierung wie in Anspruch 1 beansprucht, die umfasst:
(a) 2273 bis 2284 mg Polyethylenglycol (PEG) mit einem mittleren Molekulargewicht im Bereich von 3000 bis 4000 Da,
(b) 420 bis 446 mg Feststoff, wie z.B. Mannit,
(c) 13,5 bis 13,75 mg Magnesiumstearat und
(d) 11 bis 42 mg Aromastoff, wie z.B. Pfefferminz- oder Himbeere/Zitrone-Aroma.

17. Eine feste Formulierung nach einem vorhergehenden Anspruch zur Verwendung zur Verhinderung von Magen-Darm-Störungen oder zur Aufrechterhaltung der Magen-Darm-Gesundheit, zum Beispiel zur Verhinderung einer Austrocknung des Stuhls, zur Erweichung des Stuhls für eine leichtere Defäkation, zur Konstipationsprävention und zur Gewährleistung eines regulären gastrointestinalen Transits bei einem Subjekt (z.B. einem Menschen).

18. Eine Formulierung nach Anspruch 17, wobei das Subjekt gesund ist (d.h. einen normalen Stuhlgang besitzt).

19. Eine Formulierung nach Anspruch 18 zur Verwendung zur Verzögerung oder Verringerung der Häufigkeit von Konstipationsphasen oder zur Verhinderung der Entstehung einer Konstipation.

20. Eine feste Formulierung wie in einem der Ansprüche 1 bis 16 beansprucht, wobei das Subjekt (z.B. der Mensch) eine ausreichende Menge Formulierung einnimmt, um bis zu 6 g (oder etwa 6 g) PEG pro Tag zu erhalten.

21. Eine kaubare oder lutschbare feste Formulierung zur oralen Verabreichung als Feststoff, umfassend:
(a) 2,0 bis 3,5 g PEG (zum Beispiel 2,1 bis 2,5 g) mit einem mittleren Molekulargewicht von 3000 bis 4000 Da,
(b) 250 bis 500 mg (zum Beispiel 420 bis 450 mg) eines Feststoffs, ausgewählt aus Sorbit, Lactose, Dextraten, Cellulose, Xylit, Maltit, Mannit,
(c) gegebenenfalls Aromastoff, der, falls vorhanden, in einer Menge von 5 bis 75 mg vorliegt,
(d) gegebenenfalls Gleitmittel wie z.B. Magnesiumstearat, das, falls vorhanden, in einer Menge von 5 bis 25 mg vorliegt.

22. Die Formulierung nach Anspruch 21 zur Verhinderung einer Austrocknung des Stuhls, zur Erweichung des Stuhls für eine leichtere Defäkation, zur Konstipationsprävention, zur Gewährleistung eines regulären gastrointestinalen Transits.

23. Eine Packung, umfassend eine Mehrzahl von Einheiten aus festen Formulierungen gemäß einem der Ansprüche 1 bis 16 und 20 bis 22, zum Beispiel 5 oder mehr (wie z.B. 10), 10 oder mehr (wie z.B. 20) oder 20 oder mehr (wie z.B. 30) Einheiten der festen Formulierung.

## Revendications

1. Formulation solide pour administration orale en tant que solide, comprenant :
(a) 50 à 90 % en poids de polyéthylène glycol (PEG) ayant une masse moléculaire moyenne dans la plage de 2000 à 10 000 Da ; et
(b) 10 à 40 % en poids d'un solide choisi parmi du sorbitol, du lactose, des dextrates, de la cellulose, du xylitol, du maltitol, du mannitol.

2. Formulation solide selon la revendication 1, dans laquelle le groupe comprend en outre du lactose et de l'amidon (par exemple, un composé comprenant du lactose monohydraté et de l'amidon de maïs tel que Starlac®).

3. Formulation solide selon une quelconque revendication précédente, comprenant 82 à 84 % en poids de PEG.

4. Formulation solide selon une quelconque revendication précédente, comprenant 10 à 20 % en poids de solide du composant (b).

5. Formulation solide selon une quelconque revendication précédente, dans laquelle le solide du composant (b) est du mannitol (par exemple, du mannitol granulaire), du xylitol, du lactose et de l'amidon, ou du sorbitol.

6. Formulation solide selon la revendication 5, dans laquelle le solide du composant (b) est du mannitol, par exemple, du mannitol granulaire.

7. Formulation solide selon une quelconque revendication précédente, dans laquelle le PEG a une masse moléculaire moyenne comprise entre 3000 et 4100 Da, par exemple, dans la plage de 3000 à 4000 Da.

8. Formulation solide selon la revendication 1, comprenant :
(a) 70 à 90 % en poids de polyéthylène glycol (PEG) ayant une masse moléculaire moyenne dans la plage de 2000 à 10 000 Da ;
(b) 10 à 20 % en poids du solide tel que du mannitol ;
(c) 0 à 2,0 % en poids de lubrifiant ; et
(d) 0 à 2,0 % en poids d'arôme.

9. Formulation solide selon une quelconque revendication précédente, comprenant du PEG et du mannitol dans un rapport pondéral de PEG:mannitol = 3:1 à 9:1.

10. Formulation solide selon une quelconque revendication précédente, comprenant un lubrifiant en une quantité de 2,0 % en poids ou moins, par exemple, 0,2 à 0,8 % en poids, par exemple, 0,5 % en poids.

11. Formulation solide selon la revendication 10, dans laquelle le lubrifiant est du stéarate de magnésium.

12. Formulation solide selon une quelconque revendication précédente, comprenant un arôme.

13. Formulation solide selon une quelconque revendication précédente, qui est essentiellement dépourvue d'électrolytes (par exemple, du chlorure de sodium, du chlorure de potassium, des bicarbonates tels que le bicarbonate de sodium, des sulfates tels que le sulfate de sodium, ou des phosphates).

14. Formulation solide selon une quelconque revendication précédente qui a une masse de 0,5 à 10 g, par exemple, 1,0 à 5,0 g.

15. Formulation solide selon la revendication 1 qui a une masse de 2,0 à 3,5 g et comprend :
(a) 1,00 à 3,15 g de polyéthylène glycol (PEG) ayant une masse moléculaire moyenne dans la plage de 2000 à 10 000 Da ;
(b) 0,20 à 1,40 g de mannitol.

16. Formulation solide selon la revendication 1, qui comprend ;
(a) 2273 à 2284 mg de polyéthylène glycol (PEG) ayant une masse moléculaire moyenne dans la plage de 3000 à 4000 Da ;
(b) 420 à 446 mg d'un solide tel que du mannitol ;
(c) 13,5 à 13,75 mg de stéarate de magnésium ; et
(d) 11 à 42 mg d'un arôme, tel qu'un arôme de menthe poivrée ou de framboise/citron.

17. Formulation solide selon une quelconque revendication précédente, destinée à être utilisée pour la prévention de troubles gastro-intestinaux ou le maintien de la santé gastro-intestinale, par exemple, prévenir une déshydratation des selles, ramollir les selles pour la facilité de défécation, prévenir la constipation, permettre un transit gastro-intestinal régulier chez un sujet (par exemple, un humain).

18. Formulation selon la revendication 17, dans laquelle le sujet est en bonne santé (c'est-à-dire présente un transit intestinal normal).

19. Formulation selon la revendication 18, destinée à être utilisée pour retarder ou réduire la fréquence d'épisodes de constipation ou prévenir l'apparition de constipation.

20. Formulation solide selon l'une quelconque des revendications 1 à 16, dans laquelle le sujet (par exemple, un humain) consomme une quantité suffisante de formulation pour recevoir jusqu'à 6 g (ou à peu près) par jour de PEG.

21. Formulation solide à mâcher ou à sucer pour administration orale en tant que solide, comprenant ;
(a) 2,0 à 3,5 g de PEG (par exemple, 2,1 à 2,5 g) ayant une masse moléculaire moyenne de 3000 à 4000 Da ;
(b) 250 à 500 mg (par exemple, 420 à 450 mg) d'un solide choisi parmi du sorbitol, du lactose, des dextrates, de la cellulose, du xylitol, du maltitol, du mannitol ;
(c) un arôme facultatif qui, s'il est présent, est en une quantité de 5 à 75 mg ;
(d) un lubrifiant facultatif tel que le stéarate de magnésium, qui, s'il est présent, est en une quantité de 5 à 25 mg.

22. Formulation selon la revendication 21, destinée à être utilisée pour prévenir une déshydratation des selles, ramollir les selles pour la facilité de défécation, prévenir la constipation, permettre un transit gastro-intestinal régulier.

23. Emballage comprenant une pluralité d'unités de formulations solides selon l'une quelconque des revendications 1 à 16 et 20 à 22, par exemple, 5 ou plus (tel que 10), 10 ou plus (tel que 20) ou 20 ou plus (tel que 30) unités de la formulation solide.
